(19) Europäisches Patentamt<br>European Patent Office<br>Office européen des brevets

(11) **EP 3 590 496 A1**

(12) # EUROPEAN PATENT APPLICATION
published in accordance with Art. 153(4) EPC

(43) Date of publication:
**08.01.2020 Bulletin 2020/02**

(21) Application number: **18760310.5**

(22) Date of filing: **22.01.2018**

(51) Int Cl.:
*A61K 8/9783* *(2017.01)*　*A61K 8/06* *(2006.01)*
*A61K 8/31* *(2006.01)*　*A61K 8/34* *(2006.01)*
*A61K 8/37* *(2006.01)*　*A61K 8/39* *(2006.01)*
*A61K 8/55* *(2006.01)*　*A61K 8/67* *(2006.01)*
*A61K 8/86* *(2006.01)*　*A61K 8/891* *(2006.01)*
*A61Q 19/00* *(2006.01)*

(86) International application number:
**PCT/JP2018/001836**

(87) International publication number:
**WO 2018/159154 (07.09.2018 Gazette 2018/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(30) Priority: 28.02.2017 JP 2017036682
15.08.2017 JP 2017156782

(71) Applicant: **FUJIFILM Corporation
Tokyo 106-8620 (JP)**

(72) Inventors:
• **KANAZAWA, Katsuhiko**
**Kanagawa 258-8577 (JP)**
• **KUBO, Toshiaki**
**Kanagawa 258-8577 (JP)**
• **KINAI, Miki**
**Kanagawa 258-8577 (JP)**

(74) Representative: **Hoffmann Eitle
Patent- und Rechtsanwälte PartmbB
Arabellastraße 30
81925 München (DE)**

(54) **OIL-IN-WATER-TYPE COSMETIC**

(57) Provided is an oil-in-water type cosmetic including an evening primrose seed extract at a content of more than 0% by mass and less than 0.1% by mass with respect to a total mass of the oil-in-water type cosmetic; a carotenoid; at least one nonionic surfactant which is selected from the group consisting of a glycerin fatty acid ester, a polyglycerin fatty acid ester, a polyoxyethylene glycerin fatty acid ester, and a polyoxyethylene sorbitan fatty acid ester and in which the number of carbon atoms in the fatty acid moiety is 12 to 22; an ionic surfactant; a silicone oil having a melting temperature of 25°C or lower; an oily component having a melting temperature of higher than 25°C; and water.

EP 3 590 496 A1

Printed by Jouve, 75001 PARIS (FR)

**Description**

**BACKGROUND OF THE INVENTION**

1. Field of the Invention

The present disclosure relates to an oil-in-water type cosmetic.

2. Description of the Related Art

It is known that singlet oxygen causing photo-aging such as stains or wrinkles is generated in a case where sunlight strikes the skin.

Carotenoid is an antioxidant component derived from a natural product obtained by extraction from seaweeds, plants, or the like, exhibits an excellent ability to scavenge singlet oxygen, and is known to have an effect of suppressing photo-aging of the skin due to singlet oxygen. The carotenoid has an excellent antioxidant ability, but exhibits a poor stability. For this reason, in a case where the carotenoid is blended into a cosmetic, improvement in stability of the carotenoid has been attempted by using in combination with an antioxidant component.

For example, WO2013/002278A discloses a cosmetic including specific amounts of astaxanthin, lycopene, and tocopherol which is an antioxidant component, as a cosmetic including a carotenoid.

**SUMMARY OF THE INVENTION**

However, oxidative decomposition of the carotenoid is suppressed in a cosmetic into which an oil-soluble antioxidant component (for example, tocopherol) as a carotenoid stabilizer is blended, whereas stickiness due to the oil-soluble antioxidant component may be felt in a case where the cosmetic is applied to the skin.

One embodiment of the present invention relates to providing an oil-in-water type cosmetic including an evening primrose seed extract and a carotenoid, which has excellent stability of the carotenoid and excellent appearance stability of the formulation and exhibits reduced stickiness in a case of being applied to the skin.

Specific means for achieving the foregoing object include the following embodiments.

<1> An oil-in-water type cosmetic comprising:

an evening primrose seed extract at a content of more than 0% by mass and less than 0.1% by mass with respect to a total mass of the oil-in-water type cosmetic;
a carotenoid;
at least one nonionic surfactant which is selected from the group consisting of a glycerin fatty acid ester, a polyglycerin fatty acid ester, a polyoxyethylene glycerin fatty acid ester, and a polyoxyethylene sorbitan fatty acid ester and in which the number of carbon atoms in the fatty acid moiety is 12 to 22;
an ionic surfactant;
a silicone oil having a melting temperature of 25°C or lower;
an oily component having a melting temperature of higher than 25°C; and
water.

<2> The oil-in-water type cosmetic according to <1>, in which the carotenoid is at least one selected from astaxanthin or lycopene.
<3> The oil-in-water type cosmetic according to <1> or <2>, in which the content of the evening primrose seed extract is 0.00025% by mass to 0.025% by mass with respect to the total mass of the oil-in-water type cosmetic.
<4> The oil-in-water type cosmetic according to any one of <1> to <3>, in which a ratio of the content of the carotenoid to the content of the evening primrose seed extract is from 0.1 to 30 on a mass basis.
<5> The oil-in-water type cosmetic according to any one of <1> to <4>, in which the ionic surfactant includes an amphoteric surfactant having a double bond.

According to one embodiment of the present invention, provided is an oil-in-water type cosmetic including an evening primrose seed extract and a carotenoid, which has excellent stability of the carotenoid and excellent appearance stability of the formulation and exhibits reduced stickiness in a case of being applied to the skin.

**DESCRIPTION OF THE PREFERRED EMBODIMENTS**

Hereinafter, an example of the embodiment of the oil-in-water type cosmetic to which the present invention is applied will be described. However, the present invention is not limited to the following embodiment at all and can be implemented with appropriate modifications within the scope of the object of the embodiment of the present invention.

The numerical range indicated by using "to" in the present specification means a range including numerical values described before and after "to" as a minimum value and a maximum value, respectively.

In the numerical ranges described in a stepwise manner in the present specification, the upper limit value or the lower limit value described in a certain numerical range may be replaced with the upper limit value or the lower limit value of the numerical range in other stepwise description. In addition, in the numerical ranges described in the present specification, the upper limit value or the lower limit value described in a certain numerical range may be replaced with the values shown in the Examples.

In the present disclosure, a combination of two or more preferred aspects is a more preferred aspect.

In the present specification, unless otherwise specified, the amount of each component in the oil-in-water type cosmetic means a total amount of a plurality of substances present in the oil-in-water type cosmetic, in a case where a plurality of substances corresponding to each component are present in the oil-in-water type cosmetic.

In the present specification, the term "water phase" is used as a term contrast to the "oil phase" regardless of the type of solvent.

The term "step" in the present specification includes not only an independent step but also a step in which the intended purpose of this step can be achieved even in the case where the step cannot be clearly distinguished from other steps.

[Oil-in-water type cosmetic]

The oil-in-water type cosmetic of the present disclosure is an oil-in-water type cosmetic including an evening primrose seed extract (hereinafter, referred to as "Component A" as appropriate) at a content of more than 0% by mass and less than 0.1% by mass with respect to a total mass of the oil-in-water type cosmetic; a carotenoid (hereinafter, referred to as "Component B" as appropriate); at least one nonionic surfactant (hereinafter, referred to as "specific nonionic surfactant" or "Component C" as appropriate) which is selected from the group consisting of a glycerin fatty acid ester, a polyglycerin fatty acid ester, a polyoxyethylene glycerin fatty acid ester, and a polyoxyethylene sorbitan fatty acid ester and in which the number of carbon atoms in the fatty acid moiety is 12 to 22; an ionic surfactant (hereinafter, referred to as "Component D" as appropriate); a silicone oil (hereinafter, referred to as "Component E" as appropriate) having a melting temperature of 25°C or lower; an oily component (hereinafter, referred to as "Component F" as appropriate) having a melting temperature of higher than 25°C; and water (hereinafter, referred to as "Component G" as appropriate).

In conventional carotenoid-containing cosmetics, an oil-soluble antioxidant component (especially tocopherol) has been frequently used as a carotenoid stabilizer. However, oxidative decomposition of the carotenoid is suppressed in a cosmetic into which an oil-soluble antioxidant component as a carotenoid stabilizer is blended, whereas stickiness due to the oil-soluble antioxidant component may be felt in a case where the cosmetic is applied to the skin. Such a phenomenon tends to occur particularly in a case where a large amount of oil-soluble antioxidant component is contained to further improve the stability of carotenoids in cosmetics.

On the other hand, according to the present disclosure, it is possible to provide an oil-in-water type cosmetic which has excellent stability of the carotenoid and excellent appearance stability of the formulation and exhibits reduced stickiness in a case of being applied to the skin, by configuring the oil-in-water type cosmetic to include an evening primrose seed extract (Component A) at a content of more than 0% by mass and less than 0.1% by mass with respect to a total mass of the oil-in-water type cosmetic; a carotenoid (Component B); at least one nonionic surfactant (Component C) which is selected from the group consisting of a glycerin fatty acid ester, a polyglycerin fatty acid ester, a polyoxyethylene glycerin fatty acid ester, and a polyoxyethylene sorbitan fatty acid ester and in which the number of carbon atoms in the fatty acid moiety is 12 to 22; an ionic surfactant (Component D); a silicone oil (Component E) having a melting temperature of 25°C or lower; an oily component (Component F) having a melting temperature of higher than 25°C; and water (Component G).

In particular, in the oil-in-water type cosmetic of the present disclosure, it is considered that the combined use of the evening primrose seed extract and the carotenoid suppresses the feeling of stickiness in a case of being applied to the skin, which can be caused in a case where the oil-soluble antioxidant component is contained, while stably containing the carotenoid.

Meanwhile, since the evening primrose seed extract contains a plurality of polyphenols and also contains other plant-derived contaminants, the evening primrose seed extract has a brownish appearance. Therefore, in a case where a large amount of the evening primrose seed extract is blended in the formulation, there may occur a problem that the formulation becomes brownish. In addition, the formulation may also become colored over time due to degradation of polyphenols contained in the evening primrose seed extract. The coloring of the formulation is not suitable as a cosmetic, which is therefore undesirable.

On the other hand, the oil-in-water type cosmetic of the present disclosure is configured such that the content of the evening primrose seed extract is more than 0% by mass and less than 0.1% by mass with respect to the total mass of the oil-in-water type cosmetic. As a result, it is considered that the coloring due to the evening primrose seed extract is suppressed while stably containing the carotenoid, thus providing excellent appearance stability of the formulation.

Furthermore, the oil-in-water type cosmetic of the present disclosure is considered to have formulation suitability as an oil-in-water type cosmetic by including Component A at a content of more than 0% by mass and less than 0.1% by mass with respect to the total mass of the oil-in-water type cosmetic, Component B, Component C, Component D, Component E, Component F, and Component G.

In addition, the above speculation is not to limitedly interpret the effect of one embodiment of the present invention, but to explain it as an example.

Hereinafter, individual components of the oil-in-water type cosmetic of the present disclosure will be described in detail.

[Evening primrose seed extract: Component A]

The oil-in-water type cosmetic of the present disclosure contains an evening primrose seed extract at a content of more than 0% by mass and less than 0.1% by mass with respect to the total mass of the oil-in-water type cosmetic.

In the oil-in-water type cosmetic of the present disclosure, the evening primrose seed extract contributes to the improvement of the stability of the carotenoid which will be described later.

The evening primrose seed extract contained in the oil-in-water type cosmetic of the present disclosure can be obtained in such a manner that crushed evening primrose seeds are extracted using water, a solvent miscible with water, or a mixed solvent thereof, and the obtained extract is filtered and then dried.

The evening primrose is classified into the genus *Oenothera,* and *Oenothera laciniata, Oenothera stricta, Oenothera biennis, Oenothera erythrosepala,* and the like are known as representative species of the genus *Oenothera.*

In the oil-in-water type cosmetic of the present disclosure, the type of evening primrose is not particularly limited.

An extraction solvent for extracting an evening primrose seed extract from evening primrose seeds is not particularly limited as long as the solvent is capable of extracting the evening primrose seed extract.

Examples of the extraction solvent include water, monohydric alcohols (methanol, ethanol, and the like), polyhydric alcohols (propylene glycol, 1,3-butylene glycol, glycerin, and the like), ethyl acetate, ethyl ether, acetone, and a mixed solvent of two or more of the solvents listed above.

The evening primrose seed extract is a water-soluble component as is apparent from the extraction solvent, and has a completely different composition from evening primrose oil obtained by squeezing evening primrose seeds. The evening primrose oil is commonly known as an oil containing a large amount of $\gamma$-linolenic acid which is an unsaturated fatty acid.

The evening primrose seed extract contained in the oil-in-water type cosmetic of the present disclosure may be extracted by the above-described extraction method from a pressed cake obtained in a production process for obtaining an evening primrose oil from evening primrose seeds.

The extraction temperature is not particularly limited. For example, the extraction is carried out at a temperature of 70°C or higher.

The evening primrose seed extract may be one obtained by the above-described method or may be a commercially available product.

Examples of the commercially available product of the evening primrose seed extract include an EVENING PRIMROSE SEED EXTRACT PC (trade name, dry powder, available from Oryza Oil & Fat Chemical Co., Ltd.), an EVENING PRIMROSE SEED EXTRACT LC (trade name, available from Oryza Oil & Fat Chemical Co., Ltd.) which is an evening primrose seed extract powder dissolved in a mixed solvent of water and 1,3-butylene glycol, and LUNA WHITE B (trade name, available from ICHIMARU PHARCOS Co., Ltd.).

The evening primrose seed extract has antioxidative, antiallergic, free radical scavenging, whitening and other effects, and these effects are known to be due to polyphenols contained in the evening primrose seed extract.

As mentioned above, the evening primrose seed extract contains a plurality of polyphenols and additionally contains other plant-derived contaminants, so the appearance of evening primrose seed extract is a brown powder. Therefore, in a case where a large amount of evening primrose seed extract is blended in the formulation, a problem of coloring in brown may occur. In addition, the formulation may become colored over time also by degradation of polyphenols contained in the evening primrose seed extract.

It is considered that the carotenoid can be stably contained without the appearance of the formulation being impaired, since the coloring of the formulation due to the evening primrose seed extract is suppressed by a configuration that the oil-in-water type cosmetic of the present disclosure contains an evening primrose seed extract in an amount of more than 0% by mass and less than 0.1% by mass with respect to the total mass of the oil-in-water type cosmetic.

In addition, in a case where the content of the evening primrose seed extract in the oil-in-water type cosmetic of the present disclosure is 0.1% by mass or more with respect to the total mass of the oil-in-water type cosmetic, the improvement of the stabilizing effect of carotenoid cannot be expected. Rather, the emulsion state becomes unstable, resulting in occurrence of separation and precipitation, and therefore the formulation suitability as an oil-in-water type cosmetic is decreased.

The content of the evening primrose seed extract in the oil-in-water type cosmetic of the present disclosure is preferably 0.0001% by mass or more and less than 0.1% by mass, more preferably 0.00025% by mass or more and 0.05% by mass or less, and still more preferably 0.00025% by mass or more and 0.025% by mass or less with respect to the total mass of the oil-in-water type cosmetic.

In a case where the content of the evening primrose seed extract in the oil-in-water type cosmetic of the present disclosure is 0.0001% by mass or more with respect to the total mass of the oil-in-water type cosmetic, sufficient stabilization effect of carotenoids can be obtained.

In addition, the term "content of the evening primrose seed extract in the oil-in-water type cosmetic" as referred to in the present disclosure refers to a mass-based content of solid content of the evening primrose seed extract contained in the oil-in-water type cosmetic.

[Carotenoid: Component B]

The oil-in-water type cosmetic of the present disclosure contains a carotenoid.

The carotenoid is a pigment of terpenoids ranging from yellow to red in color, and examples thereof include carotenoids derived from plants, algae, and bacteria.

The carotenoid is not limited to a naturally occurring carotenoid, and any type of carotenoid may be used as long as it is obtained according to a common method.

Specific examples of the carotenoid include lycopene, $\alpha$-carotene, $\beta$-carotene, $\gamma$-carotene, $\delta$-carotene, actinioerythrol, bixin, *canthaxanthin,* capsorubin, $\beta$-8'-apo-carotenal (so-called apocarotenal), $\beta$-12'-apo-carotenal, xanthophylls (for example, astaxanthin, fucoxanthin, lutein, zeaxanthin, capsanthin, $\beta$-cryptoxanthin, violaxanthin, and violerythrin), and hydroxy or carboxy derivatives thereof.

Above all, from the viewpoint of high singlet oxygen scavenging ability, so-called antioxidative ability, the carotenoid is preferably at least one selected from the group consisting of astaxanthin, lycopene, and $\beta$-carotene and more preferably at least one selected from astaxanthin or lycopene. Astaxanthin, which has been shown to have an anti-inflammatory effect, an anti-skin aging effect, and a whitening effect, is particularly preferred.

(Astaxanthin)

In the present disclosure, astaxanthin includes at least one of astaxanthin or a derivative thereof (for example, an ester of astaxanthin). In the present disclosure, astaxanthin and a derivative thereof are generically referred to as "astaxanthin".

In addition to astaxanthin derived from natural products such as plants, algae, crustaceans, and bacteria, synthetic products of astaxanthin obtained in accordance with a common method may also be used as the astaxanthin.

The astaxanthin can be extracted from cultures of red yeast *Phaffia,* green algae *Haematococcus,* marine bacteria, krill, or the like.

From the viewpoint of quality and productivity, the astaxanthin is particularly preferably astaxanthin derived from an extract from *Haematococcus* algae (hereinafter, referred to as *"Haematococcus* algae extract") or an extract from krill (hereinafter, referred to as "krill extract").

Astaxanthin derived from the krill extract (astaxanthin derived from krill) can be used not only for cosmetics classified as beauty products but also for cosmetics classified as quasi-drugs.

Specific examples of the *Haematococcus* algae include *Haematococcus pluvialis, Haematococcus lacustris, Haematococcus capensis, Haematococcus droebakensis,* and *Haematococcus zimbabwiensis.*

The *Haematococcus* algae extract can be obtained in such a manner that the cell wall of the *Haematococcus* algae described above is disrupted as required according to the method disclosed in, for example, JP1993-068585A (JP-H05-068585A) and an organic solvent such as acetone, ether, chloroform, or alcohol (for example, ethanol or methanol), or an extraction solvent such as supercritical carbon dioxide is added thereto.

Examples of commercially available products of the *Haematococcus* algae extract include ASTOTS (registered trademark)-S, ASTOTS (registered trademark)-2.5 O, ASTOTS (registered trademark)-5 O, ASTOTS (registered trademark)-10 O, and the like (available from Fujifilm Corporation); ASTAREAL OIL 50F, ASTAREAL OIL 5F, and the like (available from Fuji Chemical Industries Co., Ltd.); and BIOASTIN (registered trademark) SCE7 (available from Toyo Koso Kagaku Co., Ltd).

The content of astaxanthin as true content of coloring matter in the *Haematococcus* algae extract is preferably 0.001% by mass to 50% by mass and more preferably 0.01% by mass to 40% by mass from the viewpoint of handling at the time of production.

Incidentally, the *Haematococcus* algae extract may contain astaxanthin or an ester form thereof as true content of coloring matter similarly to the coloring matter described in JP1990-049091A (JP-H02-049091A).

The *Haematococcus* algae extract containing an ester form of astaxanthin in an amount of generally 50 mol% or more, preferably 75 mol% or more, and more preferably 90 mol% or more is suitably used.

Examples of commercially available products of the krill extract include Astax-ST (trade name, available from Marine Daiou Co., Ltd.).

(Lycopene)

Lycopene is a carotenoid represented by the chemical formula $C_{40}H_{56}$ and is a red coloring agent which belongs to carotenes and exhibits an absorption maximum at 474 nm (acetone).

In lycopene, there are also cis- and trans-isomers with respect to conjugated double bonds at the center of the molecule. Examples of lycopene isomers include all-trans isomer, 9-cis isomer, and 13-cis isomer. Lycopene in the present disclosure may be any of these isomers.

Lycopene is contained in natural products such as tomato, persimmon, watermelon, and pink grapefruit and can be isolated or extracted from these natural products.

Isolates or extracts containing lycopene are commercially available in four forms: oil type, emulsion liquid type, paste type, and powder type.

In addition to lycopene derived from natural products, synthetic products of lycopene obtained according to a common method can also be used as lycopene.

One of the preferable forms of lycopene is lycopene derived from tomato.

The tomato-derived lycopene is a fat-soluble extract from tomato pulp. Lycopene contained in the fat-soluble extract from tomato pulp is particularly preferable from the viewpoint of stability, quality, and productivity.

Here, the term "fat-soluble extract from tomato pulp" refers to an extract prepared by crushing a tomato, centrifuging the crushed material to obtain a pulp-like solid, and extracting an extract from the solid using an oil solvent.

As for the fat-soluble extract from tomato pulp, a widely commercially available tomato extract as the lycopene-containing oil or lycopene-containing paste can be used.

Examples of commercially available tomato extracts include Lyc-O-Mato (registered trademark) 15% and Lyc-O-Mato (registered trademark) 6% (available from Sun Bright Co., Ltd.), and LYCOPENE 18 (available from Kyowa Hakko Kogyo Co., Ltd.)
The oil-in-water type cosmetic of the present disclosure may contain only one type of carotenoid described above or may contain two or more types thereof.

The content of the carotenoid in the oil-in-water type cosmetic of the present disclosure is not particularly limited and can be appropriately set according to the type of the carotenoid and the like.

The content of the carotenoid in the oil-in-water type cosmetic of the present disclosure is, for example, preferably 0.000001% by mass to 1% by mass, more preferably 0.000005% by mass to 0.5% by mass, and still more preferably 0.00001% by mass to 0.1% by mass with respect to the total mass of the oil-in-water type cosmetic from the viewpoint of suppression of carotenoid degradation.

The ratio of the content of the carotenoid to the content of the evening primrose seed extract in the oil-in-water type cosmetic of the present disclosure (content of carotenoid/content of evening primrose seed extract) is preferably from 0.1 to 100, more preferably from 0.1 to 75, and still more preferably from 0.1 to 30 on a mass basis.

In a case where the ratio of the content of the carotenoid to the content of the evening primrose seed extract in the oil-in-water type cosmetic of the present disclosure is within the above-mentioned range on a mass basis, the stability of the carotenoid is superior and the change in hardness of the formulation over time is further suppressed.

[Specific nonionic surfactant: Component C]

The oil-in-water type cosmetic of the present disclosure contains at least one nonionic surfactant (that is, a specific nonionic surfactant) which is selected from the group consisting of a glycerin fatty acid ester, a polyglycerin fatty acid ester, a polyoxyethylene glycerin fatty acid ester, and a polyoxyethylene sorbitan fatty acid ester, and in which the number of carbon atoms in the fatty acid moiety is 12 to 22.

In the oil-in-water type cosmetic of the present disclosure, the specific nonionic surfactant contributes to the stability of the carotenoid and the suppression of the change in hardness of the formulation over time.

Hereinafter, for convenience, glycerin fatty acid ester and polyglycerin fatty acid ester in which the number of carbon atoms in the fatty acid moiety is 12 to 22 may be generically referred to as "C-1", and polyoxyethylene glycerin fatty acid ester and polyoxyethylene sorbitan fatty acid ester in which the number of carbon atoms in the fatty acid moiety is 12 to 22 may be generically referred to as "C-2".

The glycerin fatty acid ester is not particularly limited as long as it is an ester of glycerin and a linear or branched fatty acid in which the number of carbon atoms in the fatty acid moiety is 12 to 22.

The polyglycerin fatty acid ester is not particularly limited as long as it is an ester of polyglycerin having an average degree of polymerization of 2 or more and a linear or branched fatty acid in which the number of carbon atoms in the fatty acid moiety is 12 to 22.

Examples of the linear or branched fatty acid having 12 to 22 carbon atoms include lauric acid, myristic acid, palmitic acid, stearic acid, oleic acid, linoleic acid, linolenic acid, arachidic acid, arachidonic acid, behenic acid, and isostearic acid.

Above all, C-1 is preferably at least one selected from the group consisting of glyceryl stearate, polyglyceryl-6 stearate, polyglyceryl-2 isostearate, polyglyceryl-2 stearate, polyglyceryl-10 oleate, polyglyceryl-10 stearate, polyglyceryl-6 oleate, polyglyceryl-10 isostearate, polyglyceryl-6 myristate, hexaglycerin monopalmitate, hexaglycerin monomyristate, hexaglycerin monolaurate, decaglycerin monooleate, decaglycerin monostearate, decaglycerin monopalmitate, decaglycerin monomyristic ester, and decaglycerin monolaurate, from the viewpoint of temporal stability of carotenoid and formulation; and more preferably at least one selected from glyceryl stearate or polyglyceryl-6 stearate, from the viewpoint of suppressing the change in hardness of the formulation over time.

The polyglycerin constituting the polyglycerin fatty acid ester may be linear, branched, or cyclic. The average degree of polymerization of polyglycerin is more preferably 2 or more and 10 or less.

The polyglycerin fatty acid ester is obtained, for example, by esterifying polyglycerin having an average degree of polymerization of 2 or more with a fatty acid in which the number of carbon atoms in the fatty acid skeleton is 12 to 22.

The method for producing polyglycerin fatty acid ester is not particularly limited and examples thereof include esterification of polyglycerin and fatty acid, transesterification of polyglycerin and fatty acid ester, and transesterification of polyglycerin and fats and oils.

The polyglycerin fatty acid ester may be obtained by ester bonding of one type of fatty acid to one molecule of polyglycerin, or may be obtained by ester bonding of two or more types of fatty acid to one molecule of polyglycerin.

A commercially available product can be used as C-1.

Examples of the commercially available product of C-1 include NIKKOL (registered trademark) DGMIS, NIKKOL (registered trademark) DGMS, NIKKOL (registered trademark) Hexaglyn 1-L, NIKKOL (registered trademark) Hexaglyn 1-M, NIKKOL (registered trademark) Hexaglyn 1-OV, NIKKOL (registered trademark) Decaglyn 1-L, NIKKOL (registered trademark) Decaglyn 1-M, NIKKOL (registered trademark) Decaglyn 1-SV, NIKKOL (registered trademark) Decaglyn 1-50SV, NIKKOL (registered trademark) Decaglyn 1-ISV, NIKKOL (registered trademark) Decaglyn 1-OV, and NIKKOL (registered trademark) Decaglyn 1-LN (available from Nikko Chemicals Co., Ltd.).

The polyoxyethylene glycerin fatty acid ester and the polyoxyethylene sorbitan fatty acid ester may be a compound having one ester bond in one molecule (that is, a mono fatty acid ester), a compound having two or more ester bonds in one molecule (that is, a di-fatty acid ester, a tri-fatty acid ester, or the like), or a mixture of two or more compounds in which the number of ester bonds is different.

The average addition mole number of the oxyethylene group in C-2 is not particularly limited and is, for example, preferably 2 moles to 150 moles and more preferably 6 moles to 60 moles.

Specific examples of C-2 include polyoxyethylene glyceryl isostearate, polyoxyethylene glyceryl diisostearate, polyoxyethylene glyceryl triisostearate, polyoxyethylene glyceryl oleate, polyoxyethylene glyceryl dioleate, polyoxyethylene glyceryl trioleate, polyoxyethylene sorbitan isostearate, polyoxyethylene sorbitan diisostearate, polyoxyethylene sorbitan triisostearate, polyoxyethylene sorbitan oleate, polyoxyethylene sorbitan dioleate, and polyoxyethylene sorbitan trioleate.

Above all, C-2 is preferably at least one selected from the group consisting of polyoxyethylene glyceryl isostearate, polyoxyethylene glyceryl oleate, polyoxyethylene sorbitan isostearate, and polyoxyethylene sorbitan oleate, from the viewpoint of the temporal stability of the carotenoid and the formulation; and more preferably polyoxyethylene glyceryl isostearate from the viewpoint of the temporal stability of the formulation.

A commercially available product can be used as C-2.

Examples of the commercially available product of C-2 include EMALEX GWIS-160, EMALEX GWIS-160EX, and EMALEX GWIS-260EX (available from Nihon Emulsion Co., Ltd.).

The oil-in-water type cosmetic of the present disclosure may contain only one type of specific nonionic surfactant or may contain two or more types thereof.

The content of the specific nonionic surfactant in the oil-in-water type cosmetic of the present disclosure is not particularly limited. For example, from the viewpoint of the temporal stability of the carotenoid and the formulation, the content of the specific nonionic surfactant is preferably 1% by mass or more, more preferably 3% by mass or more, and still more preferably 5% by mass or more with respect to the total mass of the oil-in-water type cosmetic.

In addition, the content of the specific nonionic surfactant in the oil-in-water type cosmetic of the present disclosure is, for example, preferably 20% by mass or less, more preferably 15% by mass or less, and still more preferably 10% by mass or less with respect to the total mass of the oil-in-water type cosmetic, from the viewpoint of the temporal stability of the carotenoid and the formulation.

[Ionic surfactant: Component D]

The oil-in-water type cosmetic of the present disclosure contains an ionic surfactant.

In the oil-in-water type cosmetic of the present disclosure, the ionic surfactant contributes to the temporal stability of the formulation.

The ionic surfactant is not particularly limited, and may be any of an anionic surfactant, a cationic surfactant, and an amphoteric surfactant.

For example, from the viewpoint of low skin irritation, the ionic surfactant is preferably an amphoteric surfactant.

The amphoteric surfactant includes an amino acid type surfactant (for example, lecithin) having an amino group as a cationic hydrophilic group, a betaine type surfactant having a quaternary ammonium salt structure, and an imidazoline type surfactant having an imidazoline ring.

From the viewpoint of the temporal stability of the formulation, it is preferable to include an amphoteric surfactant having a double bond as the amphoteric surfactant. Lecithin is more preferable as the amphoteric surfactant having a double bond.

Examples of the lecithin include various kinds of lecithins derived from plants such as soybean, corn, peanut, rapeseed, and barley; egg yolk; animals such as cattle; and microorganisms such as *Escherichia coli.*

Specific examples of the lecithin include glycerolecithins such as phosphatidic acid, phosphatidylglycerin, phosphatidylinositol, phosphatidylethanolamine, phosphatidylmethylethanolamine, phosphatidylcholine, phosphatidylserine, bisphosphatidic acid, and diphosphatidylglycerin (so-called cardiolipin); and sphingolecithins such as sphingomyelin.

In addition to lecithin having a lecithin purity of 80% or more (more preferably 90% or more), which is generally referred to as high purity lecithin, hydrogenated lecithin having no double bond (so-called hydrogenated lecithin), enzymatically decomposed lecithin, enzymatically decomposed hydrogenated lecithin, hydroxylecithin, or the like can also be used as lecithin.

A commercially available product can be used as lecithin.

Examples of the commercially available product of the lecithin include LECION (registered trademark) series (for example, LECION P (trade name)) and LECIMER EL (trade name) (available from Riken Vitamin Co., Ltd.), PHOSPHOLIPON 20 (trade name, available from Lipoid GmbH), SLP WHITE (trade name, available from Tsuji Oil Mills Co., Ltd.), and EMULMETIK 300 (trade name, available from Lucas Meyer Cosmetics S.A.S.).

The oil-in-water type cosmetic of the present disclosure may contain only one type of ionic surfactant or may contain two or more types thereof.

The content of the ionic surfactant in the oil-in-water type cosmetic of the present disclosure is not particularly limited. For example, from the viewpoint of the temporal stability of the carotenoid and the formulation, the content of the ionic surfactant is preferably 0.05% by mass or more, more preferably 0.1% by mass or more, and still more preferably 0.5% by mass or more with respect to the total mass of the oil-in-water type cosmetic.

In addition, the content of the ionic surfactant in the oil-in-water type cosmetic of the present disclosure is, for example, preferably 5% by mass or less, more preferably 3% by mass or less, and still more preferably 1% by mass or less with respect to the total mass of the oil-in-water type cosmetic, from the viewpoint of the temporal stability of the carotenoid and the formulation.

[Silicone oil having melting temperature of 25°C or lower: Component E]

The oil-in-water type cosmetic of the present disclosure contains a silicone oil having a melting temperature of 25°C or lower.

In the oil-in-water type cosmetic of the present disclosure, the silicone oil having a melting temperature of 25°C or lower contributes to the improvement of the texture of the oil-in-water type cosmetic.

Here, the term "texture of the oil-in-water type cosmetic" refers to the touch feel of the oil-in-water type cosmetic, the texture of the oil-in-water type cosmetic in a case of being applied to the skin, or the like.

The "silicone oil having a melting temperature of 25°C or lower" in the present disclosure refers to a silicone oil that is liquid at 25°C.

The silicone oil is not particularly limited as long as it is a silicone oil containing a siloxane structure and having a melting temperature of 25°C or lower.

The silicone oil is, for example, dimethicone, trisiloxane, cyclomethicone, cyclopentasiloxane, methyltrimethicone, caprylylmethicone, phenyltrimethicone, diphenyldimethicone, or diphenylsiloxyphenyltrimethicone, which has a melting temperature of 25°C or lower.

Above all, from the viewpoint that the hardness of the oil-in-water type cosmetic can be adjusted to a suitable value, it is less sticky in a case where the oil-in-water type cosmetic is applied to the skin, and a smooth feeling of use is obtained, the silicone oil having a melting temperature of 25°C or lower is preferably at least one selected from the group consisting of dimethicone, methyltrimethicone, caprylylmethicone, and cyclopentasiloxane, and more preferably dimethicone.

A commercially available product can be used as the silicone oil having a melting temperature of 25°C or lower.

Examples of the commercially available product of the silicone oil having a melting temperature of 25°C or lower include KF-96L-1.5cs, KF-96L-2cs, KF-96A-5cs, and KF-96A-6cs, which are dimethicones of Shin-Etsu Chemical Co., Ltd.

These commercially available products are preferable from the viewpoint of capable of reducing stickiness in a case where the oil-in-water type cosmetic is applied to the skin, because the feeling of use is light.

The oil-in-water type cosmetic of the present disclosure may contain only one type of silicone oil having a melting temperature of 25°C or lower or may contain two or more types thereof.

The content of the silicone oil having a melting temperature of 25°C or lower in the oil-in-water type cosmetic of the present disclosure is not particularly limited. For example, from the viewpoint of further improving the texture of the oil-in-water type cosmetic, the content of the silicone oil having a melting temperature of 25°C or lower is preferably 0.5% by mass or more, more preferably 1% by mass or more, and still more preferably 3% by mass or more with respect to the total mass of the oil-in-water type cosmetic.

In addition, the content of the silicone oil having a melting temperature of 25°C or lower in the oil-in-water type cosmetic of the present disclosure is, for example, preferably 20% by mass or less, more preferably 15% by mass or less, and still more preferably 10% by mass or less with respect to the total mass of the oil-in-water type cosmetic, from the viewpoint of obtaining preferable feeling of use.

[Oily component having melting temperature of higher than 25°C: Component F]

The oil-in-water type cosmetic of the present disclosure contains an oily component having a melting temperature of higher than 25°C.

In the oil-in-water type cosmetic of the present disclosure, the oily component having a melting temperature of higher than 25°C contributes to the suppression of stickiness in a case of being applied to the skin. In addition, the oily component having a melting temperature of higher than 25°C can also contribute to the improvement of a moisturizing feeling.

The term "oily component having a melting temperature of higher than 25°C" in the present disclosure refers to an oily component that is semi-solid or solid at 25°C.

In addition, the term "oily component" refers to a component having a solubility in water at 25°C of less than 0.1% by mass (less than 1 g/L) and commonly used as an oily component in the field of cosmetics. Specifically, fats and oils, hydrocarbon oils, waxes, fatty acids, ester oils, and the like can be exemplified.

The oily component is not particularly limited as long as it has a melting temperature of higher than 25°C. For example, from the viewpoint that the stickiness in a case of being applied to the skin is further suppressed, an oily component having a melting temperature of 35°C or higher is more preferable.

Preferred examples of the oily component having a melting temperature of higher than 25°C include vaseline (melting temperature: 36°C to 60°C), behenyl alcohol (melting temperature: 65°C to 73°C), stearyl alcohol (melting temperature: 56°C to 58°C), shea butter (melting temperature: 36°C), beeswax (melting temperature: 60°C to 65°C), white beeswax (melting temperature: 60°C to 67°C), candelilla wax (melting temperature: 66°C to 71°C), and carnauba wax (melting temperature: 80°C to 86°C).

According to these oily components, it is possible to realize an oil-in-water type cosmetic which can obtain a necessary moisturizing feeling while suppressing the stickiness in a case of being applied to the skin.

A commercially available product can be used as the oily component having a melting temperature of higher than 25°C.

Examples of the commercially available product of the oily component having a melting temperature of higher than 25°C include NIKKOL (registered trademark) Behenyl Alcohol 80 and NIKKOL (registered trademark) deodorization stearyl alcohol (available from Nikko Chemicals Co., Ltd.), NOMCOAT (registered trademark) W (available from The Nisshin OilliO Group, Ltd.), and refined candelilla wax, refined beeswax, and refined carnauba wax (available from Yokozeki Oil & Fat Industries Co., Ltd.).

The oil-in-water type cosmetic of the present disclosure may contain only one type of oily component having a melting temperature of higher than 25°C or may contain two or more types thereof.

The content of the oily component having a melting temperature of higher than 25°C in the oil-in-water type cosmetic of the present disclosure is not particularly limited. For example, from the viewpoint of being able to further suppress a feeling of stickiness in a case of being applied to the skin, the content of the oily component having a melting temperature of higher than 25°C is preferably 1% by mass or more, more preferably 2% by mass or more, and still more preferably 5% by mass or more with respect to the total mass of the oil-in-water type cosmetic.

In addition, the content of the oily component having a melting temperature of higher than 25°C in the oil-in-water type cosmetic of the present disclosure is, for example, preferably 20% by mass or less, more preferably 15% by mass or less, and still more preferably 10% by mass or less with respect to the total mass of the oil-in-water type cosmetic, from the viewpoint of obtaining preferable feeling of use.

[Water: component G]

The oil-in-water type cosmetic of the present disclosure contains water.

The water is not particularly limited, and natural water, purified water, distilled water, ion exchange water, pure water, ultrapure water (Milli-Q water or the like), and the like can be used. The Milli-Q water is ultrapure water obtained by a Milli-Q water production system of Merck Millipore, Inc.

The water contained in the oil-in-water type cosmetic is preferably purified water, distilled water, ion exchange water, pure water, or ultrapure water from the viewpoint of less impurities.

The content of water in the oil-in-water type cosmetic of the present disclosure is preferably 5% by mass or more and 99% by mass or less, more preferably 7% by mass or more and 95% by mass or less, and still more preferably 10% by mass or more and 90% by mass or less with respect to the total mass of the oil-in-water type cosmetic, from the viewpoint of obtaining an oil-in-water type form.

[Tocopherol]

The oil-in-water type cosmetic of the present disclosure can contain tocopherol.

In the oil-in-water type cosmetic of the present disclosure, the tocopherol can contribute to the improvement of the stability of carotenoids.

In the present disclosure, the tocopherol includes at least one of tocopherol or a derivative thereof (for example, an ester of tocopherol). In the present disclosure, tocopherol and a derivative thereof are generically referred to as "tocopherol".

The tocopherol is not particularly limited.

Specific examples of the tocopherol include dl-$\alpha$-tocopherol, dl-$\beta$-tocopherol, dl-$\gamma$-tocopherol, dl-$\delta$-tocopherol, dl-$\alpha$-tocopherol acetate, dl-$\alpha$-tocopherol nicotinate, dl-$\alpha$-tocopherol linoleate, and dl-$\alpha$-tocopherol succinate.

Among them, dl-$\alpha$-tocopherol, dl-$\beta$-tocopherol, dl-$\gamma$-tocopherol, dl-$\delta$-tocopherol, or a mixture thereof (so-called mix tocopherol) is preferable. In addition, acetates of these compounds are preferably used as tocopherol derivatives.

For details of tocopherol, reference can be made to the description in paragraphs [0027] to [0031] of WO2013/002278A.

A commercially available product can be used as the tocopherol.

Examples of the commercially available product of the tocopherol include RIKEN E OIL 800 (trade name, mix tocopherol, available from Riken Vitamin Co., Ltd.), dl-$\alpha$-tocopherol (available from DSM Nutrition Japan K.K.), and TOCOPHEROL 100 (available from The Nisshin OilliO Group, Ltd.).

In a case where the oil-in-water type cosmetic of the present disclosure contains tocopherol, the cosmetic may contain only one type of tocopherol or may contain two or more types thereof.

Tocopherol is known to effectively suppress the oxidative degradation of carotenoids as an antioxidant and to contribute to the stabilization of carotenoids in formulations. On the other hand, in a case where too much tocopherol is blended in anticipation of a further effect, there may occur a problem that stickiness is felt in a case of being applied to the skin.

For these reasons, in a case where the oil-in-water type cosmetic of the present disclosure contains tocopherol, the content of tocopherol in the oil-in-water type cosmetic is preferably 0.0001% by mass to 1.0% by mass and more preferably 0.003% by mass to 0.5% by mass with respect to the total mass of the oil-in-water type cosmetic.

[Other components]

The oil-in-water type cosmetic of the present disclosure may further contain other components used in cosmetics as long as the effects of the present disclosure are not impaired.

Other components include, for example, functional components that exhibit useful cosmetic effects (moisturizing effect, whitening effect, skin-adjusting effect, and the like) in a case of being used in cosmetics. Such functional components include ubiquinones such as coenzyme Q10; polysaccharides such as hyaluronic acid; glycosphingolipids such as glucosylceramide and galactosylceramide; collagens such as hydrolyzed collagen and water-soluble collagen; amino acids such as acetyl hydroxyproline; hydrolyzed white lupin protein; and ascorbyl phosphate palmitate 3Na.

Furthermore, as other components, a UV-B absorber [ethylhexyl methoxycinnamate, methylenebisbenzotriazolyl tetramethylbutylphenol (MBBT), octocrylene (2-ethylhexyl 2-cyano-3,3-diphenylprop-2-enoate), or the like], an emollient agent (cetyl ethylhexanoate, pentaerythrityl tetraethylhexanoate, or the like), a preservative (phenoxyethanol, methylparaben, ethylparaben, iodopropynyl butylcarbamate, or the like), a pH adjuster (sodium hydroxide, potassium hydroxide, hydrochloric acid, or the like), a polyhydric alcohol (1,3-butylene glycol, glycerin, or the like), a chelating agent, an antioxidant, an emulsifier, a thickener (xanthan gum or the like), a coloring agent, a flavor, and the like can be mentioned.

The oil-in-water type cosmetic of the present disclosure preferably further contains a compound that promotes the production of melatonin in the skin (hereinafter, also referred to as a "melatonin production promoter") as another component.

Melatonin is a hormone present in animals, plants, and microorganisms, and in humans it is a hormone that is mainly synthesized in the pineal body of the brain and secreted into the blood. Melatonin plays a role in controlling sleep by acting on the parasympathetic nervous system, and also plays a role in protecting nuclear DNA and mitochondrial DNA from exhibiting strong antioxidant action.

However, since melatonin cannot be directly produced in the human body, melatonin is synthesized in vivo through serotonin by several enzymatic reactions using tryptophan taken from food as a precursor.

Melatonin is also produced in organs and cells other than the above-mentioned pineal gland, an example of which is the skin. Also in the skin, it has been shown from several experiments that melatonin is produced through enzymatic reactions similar to those of the pineal gland. In addition, it has also been suggested that melatonin is involved in collagen production, and melatonin present in the skin may also be involved in the production of collagen in the skin.

However, melatonin in the pineal body may not be normally produced by various factors such as irregular sleep and aging. For this reason, promoting the production of melatonin in the skin is also considered to be effective in normalizing the amount of melatonin in the body.

Meanwhile, it is known that arylalkylamine N-acetyltransferase (AANAT) is a rate-limiting enzyme in an enzymatic reaction for producing melatonin.

Therefore, from the viewpoint as described above, in order to promote the production of melatonin in the skin, the oil-in-water type cosmetic of the present disclosure preferably contains a material that increases the amount of AANAT. Specific examples of such a material include ursolic acid and sage leaf extract.

The sage leaf extract is extracted from a sage, which is a perennial or an evergreen shrub belonging to the genus *Firmiana* of the family *Lamiaceae,* and mainly contains flavonoids, tannins, and essential oils (for example, cineole).

The sage leaf extract is preferably a sage leaf extract containing a large amount of ursolic acid, from the viewpoint of further enhancing the production of collagen in the skin.

The content of ursolic acid in the sage leaf extract is preferably 10% by mass or more, more preferably 30% by mass or more, and still more preferably 60% by mass or more, with the upper limit thereof being not particularly limited.

[Applications of oil-in-water type cosmetic]

Examples of applications of the oil-in-water type cosmetic of the present disclosure include skin care cosmetics such as milk and cream (for example, eye cream or night cream), and makeup cosmetics such as makeup base and liquid foundation. However, applications of the oil-in-water type cosmetic of the present disclosure are not limited thereto.

[Method for producing oil-in-water type cosmetic]

The method for producing the oil-in-water type cosmetic of the present disclosure is not particularly limited.

The oil-in-water type cosmetic of the present disclosure can be obtained according to a known method for producing an oil-in-water type cosmetic, using an evening primrose seed extract (Component A) at a content of more than 0% by mass and less than 0.1% by mass with respect to the total mass of the oil-in-water type cosmetic; a carotenoid (Component B); at least one nonionic surfactant (Component C) which is selected from the group consisting of a glycerin fatty acid ester and a polyglycerin fatty acid ester in which the number of carbon atoms in the fatty acid moiety is 12 to 22, and a polyoxyethylene glycerin fatty acid ester and a polyoxyethylene sorbitan fatty acid ester in which the number of carbon atoms in the fatty acid moiety is 12 to 22; an ionic surfactant (Component D); a silicone oil (Component E) which is liquid at 25°C; an oily component (Component F) which is not liquid at 25°C; water (Component G); and optionally the other components described above.

One of the preferred methods for producing the oil-in-water type cosmetic of the present disclosure is a method of mixing an oil phase composition containing Component B, Component C, Component D, Component E, and Component F with a water phase composition containing Component A and Component G, and emulsifying the mixture by a conventional method.

An example of the preferred method for producing the oil-in-water type cosmetic of the present disclosure will be described in detail.

(a) The oil phase composition is obtained by heating and mixing Component B, Component C, Component D, Component E, Component F, and optionally, other oily components.

(b) The water phase composition is obtained by heating and mixing Component A, Component G and optionally, other aqueous components.

(c) The oil phase composition and the water phase composition are mixed under stirring to effect emulsification and dispersion.

The oil-in-water type cosmetic of the present disclosure can be produced by the above method.

In the oil phase composition, Component B, Component C, Component D, Component E, Component F, and other oily components may be simply mixed, and it is preferable that those components are uniformly mixed.

Component B, Component C, Component D, Component E, Component F, and other oily components may be mixed at one time, or may be mixed while adding the other components separately to one component.

The method of mixing Component B, Component C, Component D, Component E, Component F, and other oily components is not particularly limited, and may be, for example, a method of mixing the components by stirring.

The stirring means is not particularly limited, and a common stirring instrument or stirring device can be used.

Examples of the stirring instrument or stirring device include a stirrer, a household mixer, a paddle mixer, an impeller mixer, a homomixer, a disper mixer, an ultra mixer, a high pressure homogenizer, and an ultrasonic homogenizer.

The stirring time is not particularly limited, and can be appropriately set according to the type of the stirring instrument or stirring device, the composition of the oil phase composition, and the like.

It is preferable to set the temperature at the time of mixing Component B, Component C, Component D, Component E, Component F, and other oily components to 70°C or higher from the viewpoint of solubility.

The means for adjusting the temperature is not particularly limited, and a common heating device can be used.

In the water phase composition, Component A, Component G, and other aqueous components may be simply mixed, and it is preferable that those components are uniformly mixed.

Component A, Component G, and other aqueous components may be mixed at one time, or may be mixed while adding the other components to one component in a divided manner, such as mixing the components while adding Component A and other aqueous components little by little to Component G.

The method of mixing Component A, Component G, and other aqueous components is not particularly limited, and may be, for example, a method of mixing the components by stirring.

The stirring means is not particularly limited, and a common stirring instrument or stirring device can be used. The stirring instrument or stirring device may be the same as the stirring instrument or stirring device in the preparation of the oil phase composition described above.

The stirring time is not particularly limited, and can be appropriately set according to the type of the stirring instrument or stirring device, the composition of the water phase composition, and the like.

The temperature at the time of mixing Component A, Component G, and other aqueous components is preferably set to 100°C or lower, more preferably 70°C or higher and 100°C or lower, and still more preferably 70°C or higher and 90°C or lower, from the viewpoint of preventing explosive boil.

The means for adjusting the temperature is not particularly limited, and a common heating device can be used.

The oil-in-water type cosmetic of the present disclosure can be prepared by mixing, emulsifying, and dispersing an oil phase composition and a water phase composition.

The oil phase composition and the water phase composition may be mixed at one time or may be mixed while adding one composition to the other composition little by little.

The method of mixing the oil phase composition and the water phase composition is not particularly limited, and may be, for example, a method of mixing the compositions by stirring.

The stirring means is not particularly limited, and a common stirring instrument or stirring device can be used. The stirring instrument or stirring device may be the same as the stirring instrument or stirring device in the preparation of the oil phase composition described above.

The stirring time is not particularly limited, and can be appropriately set according to the type of the stirring instrument or stirring device, the composition of the oil phase composition and the water phase composition, and the like.

The temperature of the oil phase composition at the time of mixing with the water phase composition is preferably set to 100°C or lower and more preferably 70°C or higher and 90°C or lower, from the viewpoint of preventing explosive boil.

The temperature of the water phase composition is not particularly limited, and is preferably set, for example, to 70°C or higher and 90°C or lower.

There is no particular limitation on the method of emulsification and dispersion.

For emulsification and dispersion, for example, a stirring instrument or stirring device such as a stirrer, a paddle mixer, an impeller mixer, a homomixer, a disper mixer, an ultra mixer, a homogenizer, a high pressure homogenizer, or an ultrasonic homogenizer can be used.

The time and temperature of emulsification and dispersion are not particularly limited, and can be appropriately set according to the type of the stirring instrument or stirring device, the composition of the oil phase composition and the water phase composition, and the like.

In a case where a homogenizer is used for emulsification and dispersion, for example, the rotation speed can be set to 350 revolutions per minute (rpm; the same applies hereinafter) to 7000 rpm, and the stirring time can be set to 10 minutes to 60 minutes.

The emulsification and dispersion may be carried out twice or more by changing the stirring conditions.

The ratio of the oil phase composition to the water phase composition (oil phase composition:water phase composition) is not particularly limited. For example, from the viewpoint of obtaining an oil-in-water type form, the ratio is preferably from 1:19 to 1:1, more preferably from 1:15 to 1:1.5, and still more preferably from 1:9 to 1:2 on a mass basis.

Examples

Hereinafter, the present invention will be more specifically described by way of Examples. However, the present invention is not limited to the following Examples as long as it does not depart from the gist of the present invention.

[Preparation of oil-in-water type cosmetic]

[Examples 1 to 10 and Comparative Examples 1 to 6]

The components which constitute the oil phase composition of the following Table 1 or 2 were mixed and then stirred in a water bath at 80°C for 10 minutes using a stirrer to obtain an oil phase composition. Next, the components which constitute the water phase composition of the following Table 1 or 2 were mixed and then stirred in a water bath at 80°C for 10 minutes using a stirrer to obtain a water phase composition. Then, the oil phase composition was gradually added while stirring the water phase composition in a water bath at 80°C at a rotation speed of 2500 rpm using a homomixer (model: TK ROBOMIX, available from PRIMIX Corporation). After completion of the addition, the mixture was stirred at a rotation speed of 3000 rpm for 5 minutes using a homomixer (model: TK ROBOMIX, available from PRIMIX Corporation) to obtain an emulsion. The obtained emulsion was cooled to 30°C to obtain an oil-in-water type cosmetic (cream).

[Evaluation]

The following evaluations were carried out on the oil-in-water type cosmetic of each of Examples 1 to 10 and Comparative Examples 1 to 6. The results are shown in Tables 1 and 2.

1. Formulation suitability

The formulation suitability of the oil-in-water type cosmetic immediately after preparation thereof was evaluated. Here, the formulation suitability includes both a suitability as a cosmetic (appearance and feeling of use) and a suitability as an oil-in-water type cosmetic (appearance). Further, the suitability as an oil-in-water type cosmetic means that the form as an oil-in-water type cosmetic can be well maintained.

1-1. Appearance (coloring)

The absorption spectrum of the oil-in-water type cosmetic immediately after preparation thereof was measured using a UV-visible spectrophotometer (model name: UV-2550, available from Shimadzu Corporation). Based on the obtained absorption spectrum, the appearance (coloring) of the oil-in-water type cosmetic immediately after preparation thereof was evaluated according to the following evaluation standards.

The evaluation result being "A" means that the tint derived from a carotenoid is not impaired by the formulation due to the decomposition of the carotenoid, the coloring derived from the evening primrose seed extract, or the like, and the tint as a cosmetic is satisfactory.

- Evaluation standards -

A: The absorbance (Abs) at an absorption maximum wavelength present at a wavelength of 350 nm to 380 nm is less than 0.5, and the absorbance at a wavelength of 630 nm [Abs (630 nm)] and the absorbance at a wavelength of 650 nm [Abs (650 nm)] satisfy Equation (1).

$$\text{Abs (630 nm)/Abs (650 nm)} \leq 1.05 \qquad \text{Equation (1)}$$

B: At least one of the absorbance (Abs) at an absorption maximum wavelength present at a wavelength of 350 nm to 380 nm being 0.5 or more, or Abs (630 nm) and Abs (650 nm) satisfying Equation (2) is satisfied.

$$\text{Abs (630 nm)/Abs (650 nm)} > 1.05 \qquad \text{Equation (2)}$$

1-2. Appearance (separation)

The appearance of the oil-in-water type cosmetic immediately after preparation thereof was visually observed, and the appearance (separation) of the oil-in-water type cosmetic immediately after preparation thereof was evaluated according to the following evaluation standards.

In a case where the evaluation result is "A", it means that the prepared oil-in-water type cosmetic has the suitability as an oil-in-water type cosmetic.

- Evaluation standards -

A: Separation is not confirmed at all.
B: Slight separation is confirmed.
C: Significant separation is confirmed.

1-3. Feeling of use (stickiness)

Ten expert panelists for cosmetic evaluation were asked to apply the oil-in-water type cosmetic immediately after preparation thereof on their faces with their fingers, and to judge whether or not stickiness was felt in a case where the oil-in-water type cosmetic was applied and then judge the degree of stickiness in a case where the stickiness was felt. The expert panelists were asked to score "1" in a case where no stickiness was felt at all, "2" in a case where slight stickiness was felt, and "3" in a case where stickiness was felt strongly.

The score values of individual 10 expert panelists were totaled, and the feeling of use (stickiness) was evaluated according to the following evaluation standards based on the total value thus obtained.

In a case where the evaluation result is "AA" or "A", it can be evaluated that the stickiness at the time of applying to skin is suppressed.

- Evaluation standards -

AA: The total value is 10 or more and 15 or less.
A: The total value is 16 or more and 20 or less.
B: The total value is 21 or more and 25 or less.
C: The total value is 26 or more and 30 or less.

2. Temporal stability

2-1. Stability of carotenoids

The stability over time (temporal stability) of the carotenoids (that is, astaxanthin and lycopene) contained in the oil-in-water type cosmetic was evaluated. In a case where the carotenoids contained in the oil-in-water type cosmetic are decomposed, the appearance color of the oil-in-water type cosmetic becomes thinner, so the change in appearance color was evaluated for the stability over time of the carotenoids contained in the oil-in-water type cosmetic, using the change in absorbance at a wavelength of 490 nm as an index.

First, the oil-in-water type cosmetic immediately after preparation thereof was packed in a glass base dish (AGC Techno Glass Co., Ltd.), and the absorbance at 490 nm was measured. The absorbance obtained here is referred to as "initial absorbance".

The absorbance was measured using a UV-visible spectrophotometer (model name: UV-2550, available from Shimadzu Corporation) as a measurement device under the conditions of scan speed: medium speed and slit width: 5 nm. A copper sulfate white plate was used as a reference at the time of measurement. The arrangement of the sample at the time of measurement was a reflection arrangement.

In addition, 100 g of the oil-in-water type cosmetic immediately after preparation thereof was weighed and placed in a glass bottle which was then sealed to prepare a sample for storage. Three samples for storage were prepared for each oil-in-water type cosmetic. The three prepared samples for storage were stored in thermostatic baths whose temperatures in the baths were set to 25°C, 40°C, and 50°C, respectively. Two months after storage, the samples for storage were taken out of the thermostat, and the absorbance at 490 nm of the oil-in-water type cosmetic in a glass bottle was measured by the same method as described above. The absorbance obtained here is referred to as "temporal absorbance".

The percentage change in absorbance (%) was calculated based on Equation (3) from the initial absorbance and the temporal absorbance. Then, based on the calculated value of the percentage change in absorbance (%), the stability over time of the carotenoids contained in the oil-in-water type cosmetic was evaluated according to the following evaluation standards.

$$\text{Percentage change in absorbance (\%)} = [(\text{initial absorbance-temporal absorbance})/\text{initial absorbance}] \times 100 \qquad \text{Equation (3)}$$

In addition, in consideration of the merchantability, in a case where the evaluation result is "AA" or "A", it was judged as acceptable.

- Evaluation standards -

AA: The percentage change in absorbance is less than 2%.
A: The percentage change in absorbance is 2% or more and less than 10%.
B: The percentage change in absorbance is 10% or more and less than 20%.
C: The percentage change in absorbance is 20% or more.

2-2. Formulation stability

The stability over time (temporal stability) of the oil-in-water type cosmetic was evaluated. The temporal stability of the oil-in-water type cosmetic was evaluated in terms of appearance (coloring, separation, and precipitation) and hardness.

(1) Appearance (coloring)

100 g of the oil-in-water type cosmetic immediately after preparation thereof was weighed and placed in a glass bottle which was then sealed to prepare a sample for evaluation over time. Three samples for evaluation over time were prepared for each oil-in-water type cosmetic. The three prepared samples for evaluation over time were stored in thermostatic baths whose temperatures in the baths were set to 25°C, 40°C, and 50°C, respectively. Two months after storage, the samples for evaluation over time were taken out of the thermostat, and the absorption spectra thereof were measured. For the measurement, a UV-visible spectrophotometer (model name: UV-2550, available from Shimadzu Corporation) was used. Based on the obtained absorption spectra, the temporal stability of the oil-in-water type cosmetic was evaluated in terms of appearance (coloring) according to the following evaluation standards.

The evaluation result being "A" means that the tint derived from a carotenoid is not impaired over time due to the decomposition of the carotenoid, the coloring derived from the evening primrose seed extract, or the like, and the tint as a cosmetic is well maintained.

- Evaluation standards -

A: The absorbance (Abs) at an absorption maximum wavelength present at a wavelength of 350 nm to 380 nm is less than 0.5, and the absorbance at a wavelength of 630 nm [Abs (630 nm)] and the absorbance at a wavelength of 650 nm [Abs (650 nm)] satisfy Equation (1).

$$\text{Abs (630 nm)/Abs (650 nm)} \leq 1.05 \qquad \text{Equation (1)}$$

B: At least one of the absorbance (Abs) at an absorption maximum wavelength present at a wavelength of 350 nm to 380 nm being 0.5 or more, or Abs (630 nm) and Abs (650 nm) satisfying Equation (2) is satisfied.

$$\text{Abs (630 nm)/Abs (650 nm)} > 1.05 \qquad \text{Equation (2)}$$

(2) Appearance (separation)

100 g of the oil-in-water type cosmetic immediately after preparation thereof was weighed and placed in a glass bottle which was then sealed to prepare a sample for evaluation over time. Three samples for evaluation over time were prepared for each oil-in-water type cosmetic. The three prepared samples for evaluation over time were stored in thermostatic baths whose temperatures in the baths were set to 25°C, 40°C, and 50°C, respectively. Two months after storage, the samples for evaluation over time were taken out of the thermostat, and the appearance thereof was visually observed. Then, the temporal stability of the oil-in-water type cosmetic was evaluated in terms of appearance (separation) according to the following evaluation standards.

In addition, in consideration of the merchantability, in a case where the evaluation result is "A", it was judged as acceptable.

- Evaluation standards -

A: Separation is not confirmed at all.
B: Slight separation is confirmed.
C: Significant separation is confirmed.

(3) Appearance (precipitation)

100 g of the oil-in-water type cosmetic immediately after preparation thereof was weighed and placed in a glass bottle which was then sealed to prepare a sample for evaluation over time. Three samples for evaluation over time were prepared for each oil-in-water type cosmetic. The three prepared samples for evaluation over time were stored in thermostatic baths whose temperatures in the baths were set to 25°C, 40°C, and 50°C, respectively. Two months after storage, the samples for evaluation over time were taken out of the thermostat. Each of the removed samples for evaluation over time was applied to a slide glass which was then covered with a cover glass, the sample for evaluation over time was spread out to a thickness through which light passes, and the sample for evaluation over time sandwiched between two sheets of glass was observed with an optical microscope (magnification: X1050) to confirm presence or absence and degree of precipitates. Then, the temporal stability of the oil-in-water type cosmetic was evaluated in terms of appearance (precipitation) according to the following evaluation standards.

In addition, in consideration of the merchantability, in a case where the evaluation result is "A", it was judged as acceptable.

- Evaluation standards -

A: No precipitate is confirmed at all.
B: The precipitate is slightly confirmed.
C: Precipitate is significantly confirmed.

(4) Hardness

The hardness of the oil-in-water type cosmetic immediately after preparation thereof was measured using a rheometer (model name: FUDOH RHEOMETER, available from Rheotech Co., Ltd.). The hardness obtained here is referred to as "initial hardness".
In measurement of the hardness of the oil-in-water type cosmetic, the stress from the sample in a case where a constant load was applied was detected as a load, and this load was applied as the hardness (unit: g).
Specifically, the peak value of the stress measured in a case where the tip of an adapter having a diameter of 20 mm is inserted 20 mm into each oil-in-water type cosmetic immediately after preparation thereof with a load of 200 g at a speed of 60 mm/min under the conditions of a measurement temperature of 25°C was taken as the measured value of hardness (unit: g).
In addition, 100 g of the oil-in-water type cosmetic immediately after preparation thereof was weighed and placed in a glass bottle which was then sealed to prepare a sample for evaluation over time. Three samples for evaluation over time were prepared for each oil-in-water type cosmetic. The three prepared samples for evaluation over time were stored in thermostatic baths whose temperatures in the baths were set to 25°C, 40°C, and 50°C, respectively. Two months after storage, the samples for evaluation over time were taken out of the thermostat, and the hardness thereof was measured using the above-mentioned rheometer under the same conditions as described above. The hardness obtained here is referred to as "temporal hardness".
The percentage change in hardness (%) was calculated based on Equation (4) from the initial hardness and the temporal hardness. In Equation (4), the notation "∥" represents an absolute value.
Then, based on the calculated value of the percentage change in hardness (%), the temporal stability of the oil-in-water type cosmetic was evaluated in terms of hardness according to the following evaluation standards.

$$\text{Percentage change in hardness (\%)} = [|(\text{initial hardness}-\text{temporal hardness})|/\text{initial hardness}]\times 100 \qquad \text{Equation (4)}$$

In addition, in consideration of the merchantability, in a case where the evaluation result is "AA" or "A", it was judged as acceptable.

- Evaluation standards -

AA: The percentage change in hardness is less than 5%.
A: The percentage change in hardness is 5% or more and less than 15%.
B: The percentage change in hardness is 15% or more and less than 35%.
C: The percentage change in hardness is 35% or more.

[Table 1]

| | | | Example 1 | Example 2 | Example 3 | Example 4 | Example 5 | Example 6 | Example 7 | Example 8 | Example 9 | Example 10 |
|---|---|---|---|---|---|---|---|---|---|---|---|---|
| Oil phase composition | Component B | Haematococcus algae extract (ASTOTS-S, containing 20% by mass of astaxanthin) | 0.001 | 0.03 | 0.01 | 0.01 | 0.01 | 0.01 | 0.01 | 0.01 | 0.03 | 0.01 |
| | | Tomato extract (containing 6% Lyc-O-Mato and 6% by mass of lycopene) | 0.001 | 0.025 | 0.008 | 0.008 | 0.008 | 0.008 | 0.008 | 0.008 | 0.025 | 0.008 |
| | Component C | C-1 Glyceryl stearate | 2 | 2 | 2 | 2 | 2 | 2 | 2 | 2 | 2 | 2 |
| | | C-1 Polyglyceryl-6 stearate | 1 | 1 | 1 | 1 | 1 | 1 | 1 | 1 | 1 | 1 |
| | | C-2 PEG-60 glyceryl isostearate | 2 | 2 | 2 | 2 | 2 | 2 | 2 | 2 | 2 | 2 |
| | Component D | Lecithin | 0.5 | 0.5 | 0.5 | 0.5 | 0.5 | 0.5 | 0.5 | 0.5 | 0.5 | - |
| | | Water-added lecithin | - | - | - | - | - | - | - | - | - | 0.5 |
| | Component E | Dimethicone | 2 | 2 | 2 | 2 | 2 | 2 | 2 | 2 | 2 | 2 |
| | Component F | Vaseline (melting temperature: 36°C to 60°C) | 3 | 3 | - | - | 3 | 3 | 3 | 3 | 3 | 3 |
| | | Behenyl alcohol (melting temperature: 65°C to 73°C) | 2 | 2 | 2 | 2 | 2 | 2 | 2 | 2 | 2 | 2 |
| | Other components | Cetyl ethylhexanoate | 10 | 10 | 10 | 10 | 10 | 10 | 10 | 10 | 10 | 10 |
| | | Pentaerythritol tetraethylhexanoate | 10 | 10 | 10 | 10 | 10 | 10 | 10 | 10 | 10 | 10 |
| | | Tocopherol (antioxidant) | - | - | - | - | - | - | - | - | - | - |
| Water phase composition | Component A | Evening primrose seed extract (dry powder) | 0.00025 | 0.00025 | 0.0001 | 0.00025 | 0.0025 | 0.025 | 0.05 | 0.000075 | 0.0001 | 0.0025 |
| | Other components | Glycerin (polyhydric alcohol) | 5 | 5 | 5 | 5 | 5 | 5 | 5 | 5 | 5 | 5 |
| | | 1,3-Butylene glycol (polyhydric alcohol) | 5 | 5 | 5 | 5 | 5 | 5 | 5 | 5 | 5 | 5 |
| | | Xanthan gum (thickener) | 0.15 | 0.15 | 0.15 | 0.15 | 0.15 | 0.15 | 0.15 | 0.15 | 0.15 | 0.15 |
| | | Methylparaben (preservative) | q.s. | q.s. | q.s. | q.s. | q.s. | q.s. | q.s. | q.s. | q.s. | q.s. |
| | Component G | Water | balance | balance | balance | balance | balance | balance | balance | balance | balance | balance |
| Content of Component B/Content of Component A | | | 1.0 | 30.0 | 25.0 | 9.9 | 1.0 | 0.1 | 0.05 | 33.0 | 75.0 | 1.0 |
| Total | | | 100 | 100 | 100 | 100 | 100 | 100 | 100 | 100 | 100 | 100 |
| Evaluation | Formulation suitability | Appearance (coloring) | A | A | A | A | A | A | A | A | A | A |
| | | Appearance (separation) | A | A | A | A | A | A | A | A | A | A |
| | | Feeling of use (stickiness) | A | A | AA | AA | A | A | A | A | A | A |

Temporal stability (Table 1):

| | | Example 1 | | | Example 2 | | | Example 3 | | | Example 4 | | | Example 5 | | | Example 6 | | | Example 7 | | | Example 8 | | | Example 9 | | | Example 10 | | |
|---|---|---|---|---|---|---|---|---|---|---|---|---|---|---|---|---|---|---|---|---|---|---|---|---|---|---|---|---|---|---|---|
| Aging temperature (°C) | | 25 | 40 | 50 | 25 | 40 | 50 | 25 | 40 | 50 | 25 | 40 | 50 | 25 | 40 | 50 | 25 | 40 | 50 | 25 | 40 | 50 | 25 | 40 | 50 | 25 | 40 | 50 | 25 | 40 | 50 |
| Stability of carotenoids | | AA | A | A | AA | A | A | AA | A | A | AA | AA | AA | AA | AA | A | AA | A | A | A | A | A | A | A | A | A | A | A | A | A | A |
| Stability of formulation | Appearance (coloring) | A | A | A | A | A | A | A | A | A | A | A | A | A | A | A | A | A | A | A | A | A | A | A | A | A | A | A | A | A | A |
| | Appearance (separation) | A | A | A | A | A | A | A | A | A | A | A | A | A | A | A | A | A | A | A | A | A | A | A | A | A | A | A | A | A | A |
| | Appearance (precipitation) | A | A | A | A | A | A | A | A | A | A | A | A | A | A | A | A | A | A | A | A | A | A | A | A | A | A | A | A | A | A |
| | Hardness | AA | AA | AA | AA | AA | AA | AA | AA | AA | AA | AA | AA | AA | AA | AA | AA | AA | AA | A | A | A | AA | AA | AA | AA | AA | AA | AA | A | A |

[Table 2]

| | | | Example 4 | Comparative Example 1 | Comparative Example 2 | Comparative Example 3 | Comparative Example 4 | Comparative Example 5 | Comparative Example 6 |
|---|---|---|---|---|---|---|---|---|---|
| Oil phase composition | Component B | Haematococcus algae extract (ASTOTS-S, containing 20% by mass of astaxanthin) | 0.01 | 0.01 | 0.01 | 0.01 | 0.01 | 0.01 | 0.03 |
| | | Tomato extract (containing 6% Lyc-O-Mato and 6% by mass of lycopene) | 0.008 | 0.008 | 0.008 | 0.008 | 0.008 | 0.008 | 0.02 |
| | Component C | C-1 Glyceryl stearate | 2 | 2 | 2 | 2 | 2 | 2 | 2 |
| | | C-1 Polyglyceryl-6 stearate | 1 | 1 | 1 | 1 | 1 | 1 | 1 |
| | | C-2 PEG-60 glyceryl isostearate | 2 | 2 | 2 | 2 | 2 | 2 | 2 |
| | Component D | Lecithin | 0.5 | 0.5 | 0.5 | 0.5 | - | 0.5 | 0.5 |
| | | Water-added lecithin | - | - | - | - | 1 | - | - |
| | Component E | Dimethicone | 2 | 2 | 2 | 2 | 3 | 2 | 3 |
| | Component F | Vaseline (melting temperature: 36°C to 60°C) | - | 3 | 3 | 3 | 3 | 3 | 3 |
| | | Behenyl alcohol (melting temperature: 65°C to 73°C) | 2 | 2 | 2 | 2 | 2 | 2 | 2 |
| | Other components | Cetyl ethylhexanoate | 10 | 10 | 10 | 10 | 10 | 10 | 10 |
| | | Pentaerythritol tetraethylhexanoate | 10 | 10 | 10 | 10 | 10 | 10 | 10 |
| | | Tocopherol (antioxidant) | - | - | - | - | - | 3 | 2 |
| Water phase composition | Component A | Evening primrose seed extract (dry powder) | 0.00025 | 0.1 | 1 | 2 | 2 | - | - |
| | Other components | Glycerin (polyhydric alcohol) | 5 | 5 | 5 | 5 | 5 | 5 | 5 |
| | | 1,3-Butylene glycol (polyhydric alcohol) | 5 | 5 | 5 | 5 | 5 | 5 | 5 |
| | | Xanthan gum (thickener) | 0.15 | 0.15 | 0.15 | 0.15 | 0.15 | 0.15 | 0.15 |
| | | Methylparaben (preservative) | q.s. | q.s. | q.s. | q.s. | q.s. | q.s. | q.s. |
| | Component G | Water | balance | balance | balance | balance | balance | balance | balance |
| Content of Component B/Content of Component A | | | 9.9 | 0.025 | 0.002 | 0.001 | 0.001 | - | - |
| Total | | | 100 | 100 | 100 | 100 | 100 | 100 | 100 |
| Evaluation | Formulation suitability | Appearance (coloring) | A | B | B | B | B | A | A |
| | | Appearance (separation) | A | A | B | B | B | A | A |
| | | Feeling of use (stickiness) | AA | AA | AA | AA | AA | C | B |

Temporal stability (Table 2):

| | | Example 4 | | | Comparative Example 1 | | | Comparative Example 2 | | | Comparative Example 3 | | | Comparative Example 4 | | | Comparative Example 5 | | | Comparative Example 6 | | |
|---|---|---|---|---|---|---|---|---|---|---|---|---|---|---|---|---|---|---|---|---|---|---|---|---|
| Aging temperature (°C) | | 25 | 40 | 50 | 25 | 40 | 50 | 25 | 40 | 50 | 25 | 40 | 50 | 25 | 40 | 50 | 25 | 40 | 50 | 25 | 40 | 50 |
| Stability of carotenoids | | AA | AA | AA | B | B | C | C | C | C | C | C | C | C | C | C | AA | AA | AA | AA | AA | AA |
| Stability of formulation | Appearance (coloring) | A | A | A | B | B | B | B | B | B | B | B | B | B | B | A | A | B | B | A | B | B |
| | Appearance (separation) | A | A | A | B | C | C | B | C | C | B | C | C | B | C | C | A | A | A | A | A | A |
| | Appearance (precipitation) | A | A | A | B | C | C | C | C | C | C | C | C | C | C | C | A | A | A | A | A | A |
| | Hardness | AA | AA | AA | B | C | C | C | C | C | C | C | C | C | C | C | AA | AA | AA | AA | AA | AA |

The numerical value described in the column of each component in Tables 1 and 2 indicates the content (unit: % by mass) of the component, and "-" means that the component is not contained.

In Tables 1 and 2, the "ratio of the content of Component B to the content of Component A (mass basis)" is referred to as "Content of Component B/Content of Component A".

Example 4 described in Table 2 is described for comparison with Comparative Examples, and is the same as Example 4 described in Table 1.

The details of each component described in Tables 1 and 2 are as follows.

<Component A: evening primrose seed extract>

- Evening primrose seed extract [trade name: EVENING PRIMROSE SEED EXTRACT PC, dry powder, available from Oryza Oil & Fat Chemical Co., Ltd.]

<Component B: carotenoid>

- *Haematococcus* algae extract [trade name: ASTOTS-S (containing astaxanthin: 20% by mass), available from Fujifilm Corporation]
- Tomato extract (trade name: Lyc-O-Mato (registered trademark) 6% (containing lycopene: 6% by mass), available from Sun Bright Co., Ltd.)

<Component C: specific nonionic surfactant>

(C-1)

- Glyceryl stearate (cosmetic label name, glycerin fatty acid ester in which the number of carbon atoms in the fatty acid moiety is 18)
- Polyglyceryl-6 stearate (cosmetic label name, polyglycerin fatty acid ester in which the number of carbon atoms in the fatty acid moiety is 18)

(C-2)

- PEG-60 glyceryl isostearate (cosmetic label name, polyoxyethylene glycerin fatty acid ester in which the number of carbon atoms in the fatty acid moiety is 18)

<Component D: ionic surfactant>

- Lecithin [trade name: LECION P, derived from soybean, available from Riken Vitamin Co., Ltd.]

<Component E: silicone oil having melting temperature of 25°C or lower>

- Dimethicone (cosmetic label name, trade name: KF-96A-5cs, available from Shin-Etsu Chemical Co., Ltd.)

<Component F: oily component having melting temperature of higher than 25°C>

- Vaseline (cosmetic label name, melting temperature: 36°C to 60°C)
- Behenyl alcohol (cosmetic label name, melting temperature: 65°C to 73°C)

<Other components>

- Cetyl ethylhexanoate (cosmetic label name, emollient agent)

- Pentaerythrityl tetraethylhexanoate (cosmetic label name, trade name: NS-408, available from Nippon Fine Chemical Co., Ltd., emollient agent)
- Tocopherol (trade name: RIKEN E OIL 800, mix tocopherol, available from Riken Vitamin Co., Ltd.)
- Glycerin (polyhydric alcohol)
- 1,3-Butylene glycol (polyhydric alcohol)
- Xanthan gum (thickener)
- Methylparaben (preservative)

As shown in Table 1, the oil-in-water type cosmetic of each of Examples 1 to 10, which contains an evening primrose seed extract (Component A) at a content of more than 0% by mass and less than 0.1% by mass with respect to a total mass of the oil-in-water type cosmetic; a carotenoid (Component B); at least one nonionic surfactant (Component C: specific nonionic surfactant) which is selected from the group consisting of a glycerin fatty acid ester, a polyglycerin fatty acid ester, a polyoxyethylene glycerin fatty acid ester, and a polyoxyethylene sorbitan fatty acid ester and in which the number of carbon atoms in the fatty acid moiety is 12 to 22; an ionic surfactant (Component D); a silicone oil (Component E) having a melting temperature of 25°C or lower; an oily component (Component F) having a melting temperature of higher than 25°C; and water (Component G), had excellent stability of the carotenoid and excellent appearance stability of the formulation and exhibited reduced stickiness in a case of being applied to the skin. In addition, the oil-in-water type cosmetic of each of Examples 1 to 10 also exhibited excellent hardness stability of the formulation. Furthermore, the oil-in-water type cosmetic of each of Examples 1 to 10 also exhibited excellent formulation suitability.

On the other hand, as shown in Table 2, in the oil-in-water type cosmetic of each of Comparative Examples 1 to 6, satisfactory results were not obtained in any one or more of the evaluations of the stability of the carotenoid, the appearance stability of the formulation, and the suppression of stickiness in a case of being applied to the skin.

The evaluation results of the appearance (coloring) of the formulation suitability and the stability of the carotenoid and the stability of the formulation over time were significantly inferior in the oil-in-water type cosmetic (for example, Comparative Examples 1 to 4) in which the content of the evening primrose seed extract (Component A) is 0.1% by mass or more with respect to the total mass of the oil-in-water type cosmetic, as compared with the oil-in-water type cosmetic (for example, Example 4) in which the content of the evening primrose seed extract (Component A) is less than 0.1% by mass.

The oil-in-water type cosmetic (for example, Comparative Examples 5 and 6) containing a relatively high concentration of tocopherol in place of the evening primrose seed extract (Component A) exhibited a strong feeling of stickiness in a case of being applied to the skin. In addition, the oil-in-water type cosmetic of Comparative Examples 5 and 6 showed a tendency to be inferior to the evaluation result of the stability of the formulation over time in terms of appearance (coloring) as compared with the oil-in-water type cosmetic (for example, Example 4) containing the evening primrose seed extract (Component A).

[Preparation of oil-in-water type cosmetic]

[Example 11] Cream

The following components were mixed and then stirred in a water bath at 80°C for 10 minutes using a stirrer to obtain an oil phase composition.

- Composition of oil phase composition -

| | |
|---|---|
| • *Haematococcus* algae extract | 0.01 parts by mass |
| (trade name: ASTOTS-S (containing astaxanthin: 20% by mass), available from Fujifilm Corporation) | |
| • Tomato extract | 0.008 parts by mass |
| (trade name: Lyc-O-Mato (registered trademark) 6% (containing lycopene: 6% by mass), available from Sun Bright Co., Ltd.) | |
| • Glyceryl stearate | 2 parts by mass |
| • Polyglyceryl-6 stearate | 1 part by mass |
| • PEG-60 glyceryl isostearate | 2 parts by mass |
| • Lecithin | 0.5 parts by mass |
| (trade name: LECION P, derived from soybean, available from Riken Vitamin Co., Ltd.) | |
| • Dimethicone | 2 parts by mass |

(continued)

| | |
|---|---|
| (trade name: KF-96A-5cs, available from Shin-Etsu Chemical Co., Ltd.) | |
| • Vaseline | 3 parts by mass |
| • Behenyl alcohol | 2 parts by mass |
| • Mix tocopherol | 0.52 parts by mass |
| (trade name: RIKEN E OIL 800, available from Riken Vitamin Co., Ltd.) | |
| • Cetyl ethylhexanoate | 10 parts by mass |
| • Pentaerythrityl tetraethylhexanoate | 10 parts by mass |

The following components were mixed and then stirred in a water bath at 80°C for 10 minutes using a stirrer to obtain a water phase composition.

- Composition of water phase composition -

| | |
|---|---|
| • Evening primrose seed extract | 0.0025 parts by mass |
| (trade name: EVENING PRIMROSE SEED EXTRACT PC, available from Oryza Oil & Fat Chemical Co., Ltd.) | |
| • Glycerin | 5 parts by mass |
| • 1,3-Butylene glycol | 5 parts by mass |
| • Xanthan gum | 0.15 parts by mass |
| • Methylparaben | q.s. |
| • Purified water | balance to make a total of 100 parts by mass of oil-in-water type cosmetic |

(cosmetic label name: water)

The water phase composition was stirred in a water bath at 80°C using a homomixer, and the oil phase composition was gradually added to the water phase composition under stirring. After completion of the addition, the mixture was stirred for 10 minutes at a rotation speed of 3000 rpm using a homomixer (model: TK ROBOMIX, available from PRIMIX Corporation) to obtain a crude emulsion. The resulting crude emulsion was cooled to 30°C to obtain a cream.

[Example 12] Cream

The following components were mixed and then stirred in a water bath at 80°C for 10 minutes using a stirrer to obtain an oil phase composition.

- Composition of oil phase composition -

| | |
|---|---|
| • *Haematococcus* algae extract | 0.01 parts by mass |
| (trade name: ASTOTS-S (containing astaxanthin: 20% by mass), available from Fujifilm Corporation) | |
| • Tomato extract | 0.008 parts by mass |
| (trade name: Lyc-O-Mato (registered trademark) 6% (containing lycopene: 6% by mass), available from Sun Bright Co., Ltd.) | |
| • Glyceryl stearate | 2 parts by mass |
| • Polyglyceryl-6 stearate | 1 part by mass |
| • PEG-60 glyceryl isostearate | 2 parts by mass |
| • Lecithin | 0.5 parts by mass |
| (trade name: LECION P, derived from soybean, available from Riken Vitamin Co., Ltd.) | |
| • Dimethicone | 2 parts by mass |
| (trade name: KF-96A-5cs, available from Shin-Etsu Chemical Co., Ltd.) | |
| • Behenyl alcohol | 2 parts by mass |
| • Cetyl ethylhexanoate | 10 parts by mass |

(continued)

| | |
|---|---|
| • Pentaerythrityl tetraethylhexanoate | 10 parts by mass |

The following components were mixed and then stirred in a water bath at 80°C for 10 minutes using a stirrer to obtain a water phase composition.

- Composition of water phase composition -

| | |
|---|---|
| • Evening primrose seed extract | 0.00025 parts by mass |
| (trade name: EVENING Oil & Fat Chemical Co., Ltd.) | PRIMROSE SEED EXTRACT PC, available from Oryza |
| • Sage leaf extract | 0.00025 parts by mass |
| (ursolic acid: 90% by | mass, Sabinsa Japan Corporation) |
| • Glycerin | 5 parts by mass |
| • 1,3-Butylene glycol | 5 parts by mass |
| • Xanthan gum | 0.15 parts by mass |
| • Methylparaben | q.s. |
| • Purified water | balance to make a total of 100 parts by mass of oil-in-water type cosmetic |

The water phase composition was stirred in a water bath at 80°C using a disper mixer, and the oil phase composition was gradually added to the water phase composition under stirring. After completion of the addition, the mixture was stirred for 10 minutes at a rotation speed of 3000 rpm using a homomixer (model: TK ROBOMIX, available from PRIMIX Corporation) to obtain a crude emulsion. The resulting crude emulsion was cooled to 30°C to obtain a cream.

[Example 13] Cream

The following components were mixed and then stirred in a water bath at 80°C for 10 minutes using a stirrer to obtain an oil phase composition.

- Composition of oil phase composition -

| | |
|---|---|
| • *Haematococcus* algae extract | 0.01 parts by mass |
| (trade name: ASTOTS-S (containing astaxanthin: 20% by mass), available from Fujifilm Corporation) | |
| • Tomato extract | 0.008 parts by mass |
| (trade name: Lyc-O-Mato (registered trademark) 6% (containing lycopene: 6% by mass), available from Sun Bright Co., Ltd.) | |
| • Glyceryl stearate | 2 parts by mass |
| • Polyglyceryl-6 stearate | 1 part by mass |
| • PEG-60 glyceryl isostearate | 2 parts by mass |
| • Lecithin | 0.5 parts by mass |
| (trade name: LECION P, derived from soybean, available from Riken Vitamin Co., Ltd.) | |
| • Dimethicone | 2 parts by mass |
| (trade name: KF-96A-5cs, available from Shin-Etsu Chemical Co., Ltd.) | |
| • Behenyl alcohol | 2 parts by mass |
| • Cetyl ethylhexanoate | 10 parts by mass |
| • Pentaerythrityl tetraethylhexanoate | 10 parts by mass |

The following components were mixed and then stirred in a water bath at 80°C for 10 minutes using a stirrer to obtain a water phase composition.

- Composition of water phase composition -

| | |
|---|---|
| • Evening primrose seed extract | 0.025 parts by mass |
| (trade name: LUNA WHITE B (containing evening primrose seed extract: 1% by mass), available from ICHIMARU PHARCOS Co., Ltd.) | |
| • Sage leaf extract | 0.025 parts by mass |
| (trade name: SALVIA EXTRACT, available from Maruzen Pharmaceuticals Co., Ltd.) | |
| • Glycerin | 5 parts by mass |
| • 1,3-Butylene glycol | 5 parts by mass |
| • Xanthan gum | 0.15 parts by mass |
| • Methylparaben | q.s. |
| • Purified water | balance to make a total of 100 parts by mass of oil-in-water type cosmetic |

The water phase composition was stirred in a water bath at 80°C using a disper mixer, and the oil phase composition was gradually added to the water phase composition under stirring. After completion of the addition, the mixture was stirred for 10 minutes at a rotation speed of 3000 rpm using a homomixer (model: TK ROBOMIX, available from PRIMIX Corporation) to obtain a crude emulsion. The resulting crude emulsion was cooled to 30°C to obtain a cream.

[Example 14] Whitening cream

The following components were mixed and then stirred in a water bath at 80°C for 10 minutes using a stirrer to obtain an oil phase composition.

- Composition of oil phase composition -

| | |
|---|---|
| • Astaxanthin liquid | 0.025 parts by mass |
| (Krill extract, trade name: Astax-ST (containing astaxanthin: 5% by mass), available from Marine Daiou Co., Ltd.) | |
| • Lipophilic glyceryl monostearate | 2.0 parts by mass |
| (cosmetic label name: glyceryl stearate) | |
| • Polyoxyethylene glyceryl isostearate | 2.0 parts by mass |
| • Polyglyceryl monooleate | 0.5 parts by mass |
| • Sucrose fatty acid ester | 0.5 parts by mass |
| • Soybean phospholipid | 0.5 parts by mass |
| (cosmetic label name: lecithin) (trade name: SLP WHITE, available from Tsuji Oil Mills Co., Ltd.) | |
| • Methylpolysiloxane | 2.0 parts by mass |
| (cosmetic label name: dimethicone) | |
| • Highly polymerized methylpolysiloxane (1) | 1.0 part by mass |
| (cosmetic label name: dimethicone) | |
| • Decamethylcyclopentasiloxane | 2.0 parts by mass |
| (cosmetic label name: cyclopentasiloxane) | |
| • White beeswax | 3.0 parts by mass |
| • Behenyl alcohol | 2.0 parts by mass |
| • Squalane | 10.0 parts by mass |
| • Shea butter | 5.0 parts by mass |
| Natural vitamin E | 2.0 parts by mass |
| Sodium acrylate-sodium acryloyldimethyl taurate copolymer | 2.0 parts by mass |

The following components were mixed and then stirred in a water bath at 80°C for 10 minutes using a stirrer to obtain a water phase composition.

- Composition of water phase composition -

| | |
|---|---|
| • Evening primrose seed extract | 0.025 parts by mass |
| (trade name: LUNA WHITE B (containing evening primrose seed extract: 1% by mass), available from ICHIMARU PHARCOS Co., Ltd.) | |
| • Ferulic acid | 0.01 part by mass |
| • *Centella asiatica* extract | 0.01 parts by mass |
| • Dipotassium glycyrrhizinate | 0.1 parts by mass |
| • Arbutin | 2.0 parts by mass |
| • Apricot juice | 1.0 parts by mass |
| • N-acetyl-L-hydroxyproline | 1.0 part by mass |
| • Collagen tripeptide F | 1.0 part by mass |
| • Water-soluble collagen liquid | 1.0 part by mass |
| • Hydrolyzed collagen (derived from fish) | 1.0 part by mass |
| • Sodium hyaluronate (2) | 0.5 parts by mass |
| • Glycerin | 5.0 parts by mass |
| • 1,3-Butylene glycol | 5.0 parts by mass |
| • 1,2-Pentanediol | 5.0 parts by mass |
| • Dipropylene glycol | 5.0 parts by mass |
| • Ethanol | 5.0 parts by mass |
| • Trimethylglycine | 1.0 part by mass |
| • Polyethylene glycol 6000 | 1.0 part by mass |
| • Acrylic acid-alkyl methacrylate copolymer | 0.05 parts by mass |
| • Xanthan gum | 0.15 parts by mass |
| • Phenoxyethanol | q.s. |
| • Fragrance | q.s. |
| • Purified water | balance to make a total of 100 parts by mass of oil-in-water type cosmetic |

(cosmetic label name: water)

The water phase composition was stirred in a water bath at 80°C using a disper mixer, and the oil phase composition was gradually added to the water phase composition under stirring. After completion of the addition, the mixture was stirred for 10 minutes at a rotation speed of 3000 rpm using a homomixer (model: TK ROBOMIX, available from PRIMIX Corporation) to obtain a crude emulsion. The resulting crude emulsion was cooled to 30°C to obtain a cream.

[Confirmation test of effect of sage leaf extract]

It was confirmed by the following method that the sage leaf extract contained in the oil-in-water type cosmetic of Example 12 has the effect of increasing the amount of AANAT (arylalkylamine N-acetyltransferase), which is a melatonin synthetase, and the amount of type I collagen in human skin. The sage leaf extract used was obtained from Sabinsa Japan Corporation, in which 90% by mass of ursolic acid was blended.

Normal human neonatal foreskin fibroblasts (HDF: Human Dermal Fibroblasts) were recovered from a culture flask, and then redispersed in Dulbecco's Modified Eagle's Medium (DMEM) containing 10% fetal bovine serum (FBS) to reach a cell density of 100,000 cells/mL. 1 mL/well of the cell dispersion was seeded onto a 12-well plate. Then, a test substance was added so as to be 1% dimethyl sulfoxide (DMSO) in the culture medium, followed by being dispensed to each well. Then, the plate was washed twice with 500 µL of phosphate buffered saline (PBS), and the number of viable cells was measured by a WST method. Then, 500 µL/well of a 0.1% trypsin-containing EDTA solution was added, followed by incubation at 37°C for 5 minutes, transfer to a 2 mL tube containing 500 µL of a culture medium, and neutralization. 500 µL/well of a culture medium was further added, and the remaining cells in the plate were recovered, followed by centrifugation to remove the supernatant, and then the cells were redispersed in 500 µL of PBS. After centrifugation again and removal of the supernatant, 300 µl/tube of Lysis Buffer was added to recover the cells. Next, the tube containing the recovered cells was floated on an ultrasonic cleaner and then subjected to ultrasonication for 30 minutes. This was followed by centrifugation to recover 200 µL of the supernatant in a 96-well plate.

The total amount of protein and the amount of AANAT (arylalkylamine N-acetyltransferase) were measured for the recovered supernatant. The total amount of protein was measured using a bicinchoninic acid (BCA) protein assay kit. The amount of AANAT was measured based on an enzyme-linked immunosorbent assay (ELISA). Then, the amount of AANAT was divided by the total amount of protein, and the amount of AANAT was compared between the oil-in-water type cosmetic with addition of sage leaf extract and the oil-in-water type cosmetic with no addition of sage leaf extract, assuming that the amount of AANAT in the sage leaf extract-free oil-in-water type cosmetic is taken as 100%. The amount of AANAT was increased to 111%, 114%, and 141% at 1.25 ppm, 2.5 ppm, and 5 ppm of sage leaf extract, respectively, relative to the level of AANAT in a case of no addition of the sage leaf extract.

In addition, in a case where the amount of type I collagen in the above-mentioned treated sample was measured by the ELISA method, the amount of type I collagen was increased to 114%, 132%, and 152% at 1.25 ppm, 2.5 ppm, and 5 ppm of the sage leaf extract, respectively.

From the above results, it was demonstrated that the sage leaf extract increases the amount of AANAT, which is a melatonin synthetase, and promotes the production of type I collagen.

The sage leaf extract is considered to be useful as a melatonin production promoter and as a type I collagen production promoter in human skin.

The disclosures of JP 2017-036682 filed on February 28, 2017 and JP 2017-156782 filed on August 15, 2017 are hereby incorporated by reference in their entirety.

All documents, patent applications, and technical standards described in the present specification are incorporated herein by reference to the same extent as the case where each individual document, patent application, and technical standard were specifically and individually indicated to be incorporated by reference.

**Claims**

1. An oil-in-water type cosmetic, comprising:

   an evening primrose seed extract at a content of more than 0% by mass and less than 0.1% by mass with respect to a total mass of the oil-in-water type cosmetic;
   a carotenoid;
   at least one nonionic surfactant which is selected from the group consisting of a glycerin fatty acid ester, a polyglycerin fatty acid ester, a polyoxyethylene glycerin fatty acid ester, and a polyoxyethylene sorbitan fatty acid ester and in which the number of carbon atoms in the fatty acid moiety is 12 to 22;
   an ionic surfactant;
   a silicone oil having a melting temperature of 25°C or lower;
   an oily component having a melting temperature of higher than 25°C; and
   water.

2. The oil-in-water type cosmetic according to claim 1, wherein the carotenoid is at least one selected from astaxanthin or lycopene.

3. The oil-in-water type cosmetic according to claim 1 or 2, wherein the content of the evening primrose seed extract is 0.00025% by mass to 0.025% by mass with respect to the total mass of the oil-in-water type cosmetic.

4. The oil-in-water type cosmetic according to any one of claims 1 to 3, wherein a ratio of the content of the carotenoid to the content of the evening primrose seed extract is from 0.1 to 30 on a mass basis.

5. The oil-in-water type cosmetic according to any one of claims 1 to 4, wherein the ionic surfactant comprises an amphoteric surfactant having a double bond.

## INTERNATIONAL SEARCH REPORT

International application No.

PCT/JP2018/001836

### A. CLASSIFICATION OF SUBJECT MATTER

Int.Cl. A61K8/9783(2017.01)i, A61K8/06(2006.01)i, A61K8/31(2006.01)i,
A61K8/34(2006.01)i, A61K8/37(2006.01)i, A61K8/39(2006.01)i,
A61K8/55(2006.01)i, A61K8/67(2006.01)i, A61K8/86(2006.01)i,
A61K8/891(2006.01)i, A61Q19/00(2006.01)i

According to International Patent Classification (IPC) or to both national classification and IPC

### B. FIELDS SEARCHED

Minimum documentation searched (classification system followed by classification symbols)

Int.Cl. A61K8/9783, A61K8/06, A61K8/31, A61K8/34, A61K8/37, A61K8/39,
A61K8/55, A61K8/67, A61K8/86, A61K8/891, A61Q19/00

Documentation searched other than minimum documentation to the extent that such documents are included in the fields searched

| | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)

JSTPlus/JMEDPlus/JST7580(JDreamIII)

### C. DOCUMENTS CONSIDERED TO BE RELEVANT

| Category* | Citation of document, with indication, where appropriate, of the relevant passages | Relevant to claim No. |
|---|---|---|
| Y | WO 2014/162930 A1 (FUJIFILM CORP.) 09 October 2014, claims, paragraphs [0004], [0035]–[0075], examples & EP 2982364 A1, claims, paragraphs [0006]–[0008], [0055]–[0106], examples & JP 2014-201558 A & CN 105101941 A | 1–5 |
| Y | JP 2002-308752 A (NIKKO CHEMICALS CO., LTD.) 23 October 2002, claims, paragraphs [0001], [0027]–[0035] (Family: none) | 1–5 |

☒ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 06 February 2018 (06.02.2018) | 20 February 2018 (20.02.2018) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

## INTERNATIONAL SEARCH REPORT

| International application No. |
| --- |
| PCT/JP2018/001836 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | JP 2003-128511 A (ICHIMARU PHARCOS CO., LTD.) 08 May 2003, claims, paragraphs [0012]-[0017], examples (Family: none) | 1, 3, 5 |
| A | JP 2001-335497 A (KOSÉ CORPORATION) 04 December 2001, entire text (Family: none) | 1-5 |
| A | JP 2004-137233 A (KOSÉ CORPORATION) 13 May 2004, entire text (Family: none) | 1-5 |
| A | JP 2001-233725 A (SHIN NIPPON YAKUGYO CO., LTD.) 28 August 2001, entire text (Family: none) | 1-5 |
| A | JP 2003-261435 A (ICHIMARU PHARCOS CO., LTD.) 16 September 2003, entire text (Family: none) | 1-5 |
| A | JP 2006-117612 A (ICHIMARU PHARCOS CO., LTD.) 11 May 2006, entire text (Family: none) | 1-5 |
| A | BIRCH, et al., "Antioxidant Properties of Evening Primrose Seed Extracts", J Agric Food Chem, September 2001, vol. 49, no. 9, pp. 4502-4507 | 1-5 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013002278 A
- JP 5068585 A
- JP H05068585 A
- JP 2049091 A
- JP H02049091 A
- JP 2017036682 A
- JP 2017156782 A